# EUROPÄISCHE PATENTSCHRIFT

(11) **EP 2 307 463 B1**
(45) Veröffentlichungstag und Bekanntmachung des Hinweises auf die Patenterteilung: **27.06.2012**
(21) Anmeldenummer: 09777372.5
(22) Anmeldetag: 23.07.2009
(51) Int. Cl.: C08F 10/00, B65D 81/00, A61K 8/31, A61K 8/37, A61K 8/81, A61Q 15/00

(54) **ÖLMIGRATIONSINHIBITIERTES PRODUKT**
OIL MIGRATION INHIBITED PRODUCT
PRODUIT INHIBITEUR DE LA MIGRATION DE L'HUILE

(30) Priorität: 25.07.2008 DE 102008034957
(43) Veröffentlichungstag der Anmeldung: 13.04.2011
(73) Patentinhaber: Beiersdorf AG, 20253 Hamburg (DE)
(72) Erfinder: URBAN, Michael, 22523 Hamburg (DE); HETZEL, Frank, 21261 Welle (DE); LONGREÉ, Inka, 22085 Hamburg (DE); MÜLLER, Claudia, 25499 Tangstedt (DE); KUX, Ulrich, 22559 Hamburg (DE); SCHOLZ, Annika, 22459 Hamburg (DE)
(74) Vertreter: Hartmann, Jost
(86) Internationale Anmeldenummer: PCT/EP2009/005330
(87) Internationale Veröffentlichungsnummer: WO 2010/009880

(56) Entgegenhaltungen:
- EP-A1- 1 618 925
- WO-A2-2005/058586

## Beschreibung

Die Erfindung umfasst Produkte umfassend Packmittel bestehend aus oder umfassend Polyolefine und darin enthaltende Emulsionszubereitungen, die Lipide und einen Migrationsinhibitor umfassen. Die Migrationsinhibitoren werden gewählt aus der Gruppe bestehend aus Paraffinöl, hydrogenated Polydecen und/oder Decyloleat.

In der Kosmetik- und Lebensmittelindustrie sind Verpackungen auf Polyolefinbasis bekannt. Bekannteste Vertreter der Polyolefine sind Polypropylene (PP) und Polyethylene (PE), die als Verpackungsmaterial einer der meistgebrauchten Kunststoffe sind. Sie lassen sich zu starren und flexiblen Packmitteln verarbeitet oder als Beschichtung einsetzen.
Häufig werden gespritzte oder tiefgezogene Behälter für das Verpacken eingesetzt. PP hat wie die Polyethylene eine sehr niedrige Wasserdampfdurchlässigkeit und eignet sich wegen seines hohen Schmelzpunktes zur Heißabfüllung oder sogar zur Sterilisation. PP-Packungen können, wie alle Polyolefin-Packungen, sicher versiegelt werden. Für die Verwendung für Tiefkühlkost, Eiscreme, Desserts und Molkereiprodukte werden häufig Copolymere aus Ethylen und Propylen eingesetzt.

Hauptanwendungsgebiete der PE Verpackungen sind Hohlkörper sowie Transportgefäße (Kanister, Fässer), Lagertanks und Spritzgußteile (Flaschenkästen). Aus LDPE werden Tuben und Behälter hergestellt. Weitere Einsatzbeispiele sind Folien für Säcke, dünne Folien für Beutel und Tragetaschen. Bei Coextrusion einer weißpigmentierten, mit einer braun eingefärbten LDPE-Folie wird PE zur Herstellung von Trinkmilchbeuteln verwendet. Die Verarbeitung von Polypropylen und Polyethylen erfolgt meist durch Spritzgießen, Tiefziehen und Extrusion zu Folien.

PP zeichnet sich durch hohe Härte, Rückstellfähigkeit, Steifheit und Wärmebeständigkeit aus. Kurzfristiges Erwärmen von Gegenständen aus PP ist sogar bis 140°C möglich. Bei Temperaturen unter 0°C tritt eine gewisse Versprödung der PP ein, die jedoch durch Copolymerisation des Propylens mit Ethylen (EPM, EPDM) zu wesentlich tieferen Temperaturbereichen verschoben werden kann. Allgemein lässt sich die Schlagzähigkeit von PP durch Modifikation mit Elastomeren verbessern. Die Chemikalienbeständigkeit ist wie bei allen Polyolefinen gut. Eine Verbesserung der mechanischen Eigenschaften der PP erreicht man durch Verstärkung mit Talkum, Kreide, Holzmehl oder Glasfasern, und auch das Aufbringen metallischer Überzüge ist möglich. PP sind in noch stärkerem Maße als PE oxidations- und lichtempfindlich, weshalb der Zusatz von Stabilisatoren (Antioxidantien, Lichtschutzmittel, UV-Absorber) erforderlich ist. Andererseits können PP durch Zusatz von Metalldialkyldithiocarbamaten photochemisch abbaubar gemacht werden.
Die chemisch-physikalischen Eigenschaften der PE werden durch ihren Charakter als partiell kristalline Kohlenwasserstoffe bestimmt. PE sind bis zu 60°C in allen üblichen Lösungsmitteln praktisch unlöslich. Aliphatische und aromatische Kohlenwasserstoffe und deren Halogen-Derivate bewirken erhebliche Quellung, polare Flüssigkeiten wie Alkohole, Ester und Ketone bei 20°C dagegen kaum Quellung. Gegen Wasser, Laugen und SalzLösungen sowie anorganische Säuren, mit Ausnahme der stark oxidierenden, verhalten sich PE völlig indifferent. Bei HDPE liegt die obere Gebrauchstemperatur bei ca.100°C, von LDPE bei ca. 75°C. Der geringe Schmelzpunkt von LDPE steigt mit zunehmender Dichte von 104 auf 120°C. In ungefärbtem Zustand und in Abhängigkeit von ihrer Dicke sind PE milchig weiß bis transparent opak. Bei sehr dünnen Folien ist eine fast glasklare Einstellung möglich. PE ist zäh, elastisch und kältefest bis ca. -50°C
Polyethylene haben eine sehr geringe Wasserdampfdurchlässigkeit, die Diffusion von Gasen sowie von Aromastoffen und ätherischen Substanzen durch PE ist dagegen relativ hoch. PE sind geschmacksfrei, geruchlos, physiologisch unbedenklich und für den Kontakt mit Lebensmitteln geeignet.
Zu den genannten Kurzzeichen ist anzumerken, dass auch eine andere Schreibweise für sie verwendet wird, die die Buchstaben PE voranstellt (PE-LD, PE-LLD, PE-HD, PE-HD-MHW, PE-HD-UHMW, PE-MD, PE-VLD).

Auf dem Gebiet der Kosmetik liegen breite Vorbeschreibungen hinsichtlich Emulsionen und deren Bestandteile vor, exemplarisch sei auf WO 1996028131 A2 und WO 1996028132 A2 verwiesen, die kosmetische Mikroemulsionen beschreiben.
Ebenfalls werden Antitranspirantprodukte auf Basis von Emulsionen umfassend ein oder mehrere Lipide beschrieben, wie in DE 10149373 A1.

Es zeigt sich jedoch, dass im Stand der Technik wenig zu der Kompatibilität der unterschiedlichen kosmetischen Zubereitungen und den einzelnen Packmittel-Materialien, insbesondere auf Polyolefinbasis, eingegangen wird.

Wie die Ausführungen zu Polyolefin-Verpackungen zeigen, ist die Chemikalienbeständigkeit bei allen Polyolefinen gut, jedoch beispielsweise die Diffusion von Gasen sowie von Aromastoffen und ätherischen Substanzen durch PE relativ hoch. Ebenso ist eine Quellung bei PE Verpackungen bei Kontakt mit aliphatischen oder aromatischen Kohlenwasserstoffen zu beobachten.

Es zeigt sich, dass es Probleme bei der Verpackung von lipidhaltigen kosmetischen Zubereitungen in Polyolefinverpackungen gibt.
So sind beispielsweise kosmetische Deodorantien oder Antitranspirantien in Form von Mikroemulsionen nicht mit polyolefinischen Packmitteln, insbesondere Polypropylen-Packmitteln, kompatibel.
Die in diesen beispielhaften Deo-/AT-Zubereitungen enthaltene Lipidkomponente Dicaprylylether diffundiert mit der Zeit durch das PP-Packmittel und bewirkt so eine Verölung der äußeren Packmitteloberfläche und das Ablösen von Etiketten.

Dieser Missstand führte erfindungsgemäß zu einer näheren Betrachtung des Problems. Es wurde festgestellt, dass eine Reihe von in Kosmetika häufig verwendeten Ölen durch polyolefinische Packmittel, insbesondere Polypropylen-Packmittel, hindurch migrieren. D.h. nach längerer Lagerung treten Öle an der äußeren Oberfläche aus und machen das Packmittel unansehnlich.
Weiterhin wird aufgrund der Öl-Migration eine Eintrübung des Füllgutes und physikalischen Instabilitäten (Aufrahmung) der kosmetischen Zubereitung beobachtet.

In Folge der Öl-Migration sind außerdem erhöhte Gewichtsverluste nach Langzeitlagerung der Zubereitungen zu beobachten und infolge der vorbeschriebenen Effekte kommt es ferner zu einem starken Parfümgeruch durch das polyolefinische Packmittel hindurch, da auch bestimmte Parfümöle diesen Migrationseffekt durch polyolefinische Materialien zeigen oder aufgrund ihres lipophilen Charakters vom migrierenden Lipid mitgeschleppt werden.

Lipide als Oberbegriff für Öle und Fette unterscheiden sich unter anderem in ihrer Polarität. Es wurde vorgeschlagen, die Grenzflächenspannung gegenüber Wasser als Maß für den Polaritätsindex eines Öls bzw. einer Ölphase anzunehmen. Dabei gilt, dass die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen. Als polar werden Lipide angesehen, deren Grenzflächenspannung gegen Wasser weniger als 20 mN/m beträgt, als unpolar solche, deren Grenzflächenspannung gegen Wasser mehr als 30 mN/m beträgt. Lipide mit einer Grenzflächenspannung gegen Wasser zwischen 20 und 30 mN/m werden im Allgemeinen als mittelpolar bezeichnet.

Als Abhilfe dieser Packmittelinkompatibilität bestimmter Lipide käme für den Fachmann zunächst ein Austausch der Lipidkomponente in Betracht. Um mögliche Wechselwirkungen zwischen Füllgut und polyolefinischem Packmittel zu minimieren, lag dabei - gemäß dem bekannten Paracelsus'schen Grundsatz "Gleiches löst sich in Gleichem" - die Suche nach polareren Lipidkomponenten nahe, da entsprechend dem vorgenannten Prinzip zu erwarten war, dass ein Packmittel aus einem unpolaren Material wie PP die Migration unpolarer Lipidkomponenten fördert.

Unter Berücksichtigung der aktuell einsetzbaren Lipiden mit geringer Polarität (hohen Grenzflächenspannungswerten > 30 mN/m z.B. Dicaprylyl Ether, Dicaprylyl Carbonate, lag die Suche nach mittel- bis hoch-polaren Alternativlipiden (Grenzflächenspannungswerten < 30 mN/m) nahe.
Denkbar wären so beispielsweise Formulierungen enthaltend Lipide mittlerer Polarität wie z.B. die Fettsäureester Coco-Caprylate/Caprate, Isopropyl Stearate oder Isopropyl Palmitate.
In Untersuchungen zeigte sich aber, dass auch diese Alternativlipide nicht die gewünschte Kompatibilität mit polyolefinischen Packmitteln aufweisen.
Vielmehr zeigen die Lipide C13-16 Isoparaffin (39,5), Dicaprylyl Ether (30,9), Dicaprylyl Carbonate (31,7), Diethylhexyl Carbonate, Coco-Caprylate/Caprate (23,1), C12-15 Alkyl Benzoate (21,8), Ethylhexyl Cocoate (30,0) und Isopropyl Stearate (21,9), Octyldodecanol, Isopropyl Palmitate (IPP), Isopropyl Myristate (IPM), Triethyl Citrate (TEC) eine Migration durch polyolefinische, insbesondere Polypropylen, Packmittel. In Klammern sind die Polaritäten der Lipide in mN/M angegeben.

Auffällig ist der große Anteil von "Ester-Ölen" in der Liste der mit polyolefinischen Packmitteln inkompatiblen Lipiden. Dass Ester stark mit manchen Kunststoffen interagieren ist aus dem Kfz-Bereich bekannt, wo RME (Rapsölmethylester, sog. "Biodiesel) zu Problemen mit Dichtungen und Leitungen führt.
Wünschenswert ist es daher kosmetische Zubereitungen bereit zu stellen, die trotz der angeführten Probleme in polyolefinischen Packmitteln stabil und langfristig verpackt werden können.

Einfach auf die kritischen Öle zu verzichten ist nicht möglich, da hohe Ölgehalte aufgrund ihrer hautpflegenden Eigenschaften als vorteilhaft angesehen werden. Weiterhin sind Öle in der Lage weiße Rückstände von z.B. Antitranspirant-Produkten, zu kaschieren und damit optisch zu minimieren.

Weiterhin ist es wünschenswert klare bis transluzente Formulierungen bereit zu stellen, die trotz hoher Ölgehalte eine gute Kompatibilität mit polyolefinischen Packmitteln, insbesondere aus Polypropylen, aufweisen und eine gute kosmetische Akzeptanz in Verbindung mit physikalischer Stabilität gewährleisten, d.h. z.B. keine Phasenseparation aufweisen.
Gefordert wird weiterhin auch eine sehr gute Elektrolyt-Toleranz der Zubereitungen in Kombination mit dem Packmittel um die Formulierungsfreiheit und geforderte physikalische Stabilität zu gewährleisten.

Wünschenswert für Deo/AT-Anwendungen sind insbesondere Roll-on fähige Formulierungen mit einer bestimmten Viskosität.

Wünschenswert ist es weiterhin kosmetische Zubereitungen, insbesondere auf Emulsionsbasis, mit einem Parfümgehalt stabil und lagerfähig herzustellen.

Überraschend wurde gefunden, dass die Lösung der angeführten Probleme in dem Zusatz von Migrationsinhibitoren in lipidhaltige Zubereitungen liegt.

Das erfindungsgemäße Produkt umfasst daher Packmittel bestehend aus oder umfassend Polyolefine und im Packmittel enthaltende Emulsionszubereitungen umfassend mindestens einen Migrationsinhibitor und mindestens ein Lipid, welches ohne Vorhandensein des Migrationsinhibitors in oder durch das polyolefinische Packmittel migriert.

Die Migrationsinhibitoren werden dabei gewählt aus der Gruppe umfassend Paraffinöl, hydrogenated Polydecen und/oder Decyloleat.

Die Migrationsinhibitoren werden Zubereitungen zugesetzt, die in polyolefinischen Packmitteln, insbesondere Polypropylen-Packmittel, aufbewahrt werden.

Erfindungsgemäß werden ein oder mehrere Migrationsinhibitoren, gewählt aus der Gruppe Paraffinöl, hydrogenated Polydecen und/oder Decyloleat, in Zubereitungen verwendet um die Migration von Lipiden gewählt aus der Gruppe C13-16 Isoparaffin, Dicaprylyl Ether, Dicaprylyl Carbonate, Diethylhexyl Carbonate, Coco-Caprylate/Caprate, C12-15 Alkyl Benzoate, Ethylhexyl Cocoate, Isopropyl Stearate, Octyldodecanol, Isopropyl Palmitate, Isopropyl Myristate und/oder Parfümöle, insbesondere umfassend Triethyl Citrate, Dipropylenglycol und/oder Hexylsalicylat, in oder durch das polyolefinische Packmittel zu vermindern oder zu vermeiden.

So wurde bei einer kosmetischen Zubereitung umfassend u.a. 3 Gew.% Dicaprylyl Ether und 3 Gew.% Octyldodecanol in einem PP-Packmittel die zuvor geschilderte Ölmigration, insbesondere des Dicaprylyl Ether, durch das Packmittel beobachtet.
Durch Austausch der 3 % Dicaprylyl Ether gegen 3 % Paraffinöl und ansonsten Beibehaltung der sonstigen Bestandteile der Zubereitung wurde überraschend keine Öl-Migration mehr beobachtet. D.h. die aufgrund der Polarität zu erwartende Migration des Paraffinöls und/oder Octyldodecanols fand nicht statt.

In einer weiteren Untersuchung wurde bei einem Deo/AT-Produkt in einem PP-Packmittel, das 4,5 Gew.% C13-16 Isoparaffin und 4,5 Gew.% Paraffinöl enthält, trotz des an sich PP-inkompatiblen C13-16 Isoparaffins, keine Migration des Isoparaffins beobachtet.

Der Zusatz von Migrationsinhibitoren, insbesondere Paraffinöl, zu Zubereitungen, enthaltend in oder durch polyolefinische Packmittel migrationsfähige Öle gewählt aus C13-16 Isoparaffin, Dicaprylyl Ether, Dicaprylyl Carbonate, Diethylhexyl Carbonate, Coco-Caprylate/Caprate, C12-15 Alkyl Benzoate, Ethylhexyl Cocoate, Isopropyl Stearate, Octyldodecanol, Isopropyl Palmitate, Isopropyl Myristate und/oder Parfümöle, insbesondere umfassend Triethyl Citrate, Dipropylenglycol und/oder Hexylsalicylat, verhindert oder vermindert zumindest deren Migration.

Ebenso ist es erfindungsgemäß, Öle, die in oder durch das polyolefinische Packmittel migrieren können, durch die Migrationsinhibitoren, insbesondere Paraffinöl, hydrogenated Polydecene und/oder Decyloleat, zu ersetzen. Dann umfasst die Zubereitung, vorteilhaft eine Mikroemulsion, neben den Migrationsinhibitoren ggf. nur Parfümöle als Lipidkomponenten.

Vorteilhaft können diese migrationsfähigen Öle vollständig durch den Migrationsinhibitor, insbesondere Paraffinöl, ersetzt werden und sind dennoch erfindungsgemäß als eine anwendungsfähige Zubereitung, beispielsweise als Antitranspirantmittel, formulierbar.

Somit stellen Zubereitungen in polyolefinischen Packmitteln, die ein oder mehrere Migrationsinhibitoren umfassen und kein migrationsfähiges Öl, insbesondere kein Öl gewählt aus C13-16 Isoparaffin, Dicaprylyl Ether, Dicaprylyl Carbonate, Diethylhexyl Carbonate, Coco-Caprylate/Caprate, C12-15 Alkyl Benzoate, Ethylhexyl Cocoate, Isopropyl Stearate, Octyldodecanol, Isopropyl Palmitate und/oder Isopropyl Myristate eine spezielle erfindungsgemäße Ausführungsform dar.
Diese Zubereitungen, vorteilhaft als Mikroemulsion formuliert, umfassen als Lipidkomponenten neben den Migrationsinhibitoren dann ggf. nur Parfümöle.

Erfindungsgemäß ist der Migrationsinhibitor in der Zubereitung enthalten.
Eine andere Lösung des Problems wäre die Beschichtung des Packmittels, so dass eine Migration nicht stattfinden kann. Dies ist erfindungsgemäß nicht umfasst und stellt zudem eine kostspieligere Variante dar. Das Packmittel ist auf der mit der Zubereitung in Kontakt tretenden Fläche daher vorzugsweise nicht beschichtet oder laminiert. Beispielsweise ist bekannt, die Innenflächen der Polyolefinpackmittel zu laminieren oder zu beschichten um ein Durchdringen, Migrieren, der Bestandteile zu verhindern. Auf diese Beschichtungsmaßnahmen kann nun erfindungsgemäß verzichtet werden.

Bestimmte Kosmetika sind auf einen hohen Ölgehalt angewiesen. So gibt es kosmetische Zubereitungen des Standes der Technik, die einen Ölgehalt > 6 % bei einem Emulgatorgehalt von 6 % umfassen. Derartige Formulierungen besitzen eine transluzente Optik, d.h. eine Transmission (420 nm) von ca. 2 - 6 %, wie auch erfingdungsgemäß erwünscht. Die mittlere Tröpfchengröße der Emulsion liegt bei ca. 50 nm, es handelt sich also um eine Mikroemulsion.

Nachteilig zeigt sich auch hier die nicht vorhandene Kompatibilität zu polyolefinischen Packmitteln, welche aufgrund der nochmals gesteigerten Lipidkonzentration die eingangs beschriebenen Probleme noch verstärkt.

Auch hier liegt die Lösung im Zusatz von Migrationsinhibitoren, welche überraschend auch hier vorzugsweise unpolare Eigenschaften besitzen, wie Paraffinöle. Es wurde festgestellt, dass Formulierungen die einen Ölgehalt > 6 % bei einem Emulgatorgehalt von 6 % durch teilweise Formulierung mit dem sehr unpolaren Paraffinöl auch die anteilige Formulierung mit Lipiden höherer Polarität ermöglicht. Im konkreten Beispiel konnten so Rezepturen mit Beimischungen von Guerbet-Alkoholen, vorzugsweise Octyldodecanol bei gleichzeitiger Polypropylen-Packmittelkompatiblität realisiert werden, obwohl Octyldodecanol, wie zuvor getestet, durch PP migrieren kann.

Paraffinöl, auch als Paraffinwachs oder Weißöl, E 905 bezeichnet, ist ein festes oder flüssiges Gemisch gereinigter, gesättigter aliphatischer Kohlenwasserstoffe (Paraffine). DAB 10 beschreibt zwei sogenannte medizinische Weißöle, das dickflüssige Paraffinum liquidum und das dünnflüssige Paraffinum perliquidum.
Die flüssigen Paraffinöle werden auch als Mineralöle bezeichnet und sind als Weichparaffin oder Petrolatum in Gebrauch.

Hydrogenated polydecene, IUPAC Name: dec-1-ene mit der CAS Number 68037-01-4, ist ein synthetisches Polymer, das in der Kosmetik eingesetzt wird.

Decyloleat ist ein Ester der Ölsäure. Oleat ist eine Sammelbezeichnung für Salze der Ölsäure und für Ölsäureester. Die meist als Oleate der Alkohole (beispielsweise Butyl-, Ethyl-, Oleyloleat) benannten Ester der Ölsäure sind im allgemeinen farblose bis gelbliche, relativ hochsiedende, ölige Flüssigkeiten, die in Wasser unlöslich, in organischen Lösemitteln und pflanzlichen und mineralischen Ölen löslich und z.B. als Schmiermittel einsetzbar sind. Da Ölsäureester leicht in die Haut eindringen und nicht fetten, können sie in kosmetischen Zubereitungen eingesetzt werden Paraffinöle haben eine Polarität von 43,7, Hydrogenated Polydecene von 46,7 und Decyloleate von 18,3 mN/m.

Probleme der Ölmigration werden insbesondere bei emulsionsbasierten kosmetischen Zubereitungen, wie beispielsweise zuvor erwähnte Deo-/AT-Formulierungen, beobachtet. Insbesondere wenn es sich um Mikroemulsionen handelt.

Mikroemulsionen umfassen häufig kritische Esteröle, die in Roll-on Verpackungen zu einer Quellung von PP-haltigen Kugel und Fitment führen und somit zu blockierten Kugeln führen können. Ferner sind Instabilitäten der Formelmatrix infolge von Ölverlusten möglich. Auch diese Probleme werden erfindungsgemäß gelöst.

Weiterhin wurde gefunden, dass es bei Verwendung der Migrationsinhibitoren, insbesondere Paraffinöl und hydrogenated Polydecene, vorteilhaft ist, den HLB-Wert der verwendeten Emulgatormischung der Zubereitung leicht anzuheben.
Beispielsweise zeigte sich, dass bei einer Emulgatorenmischung von 4,8 % Isoceteth-20 (HLB 15,7) und 2,4 % Gylceryl Isostearate (HLB 5,0) eine Änderung auf 5,0% Isoceteth-20 und 2,2 % Gylceryl Isostearate sich in Kombination mit Paraffinöl eine verbesserte Stabilität der Zubereitung zeigte, die diese Emulgatormischungen enthält.

Weiterer Vorteil ist, dass trotz des Zusatzes an Paraffinölen durch diese Emulgatorauswahl (Erhöhung des HLB-Wertes) die kosmetischen Zubereitungen transparent formuliert werden können. Transparent wird dabei definiert als Transmission [420 nm] > 70 %. Die Aufgabe der Formulierungsfreiheit ist damit trotz des Zusatzes an Paraffinölen gegeben.

Die erfindungsgemäßen Zubereitungen als Emulsion, insbesondere Mikroemulsion, lassen sich vorteilhaft durch PEG-modifizierte Distearate, insbesondere durch PEG-150 bis PEG-250 Distearat transparent verdicken, da die hydrophoben Stearat-Reste eine geeignete Affinität zu den dispergierten Öltröpfchen besitzen. Aus einer nicht vorhandenen Kompatibilität resultieren negative Effekte wie Eintrübung, mangelhafte Verdickung und physikalische Instabilitäten. Solche Effekte sind beispielsweise bei der Verdickung von Silikonöl-basierenden Systemen mit den beschriebenen PEG-Distearaten zu beobachten.

Bevorzugt sind die erfindungsgemäßen Zubereitungen auf Emulsionsbasis, insbesondere Mikro- oder Nanoemulsionen, insbesondere feinst-disperse Systeme mit Tröfpchengröße < 100 nm, besonders bevorzugt mit einer Tröpfchengröße zwischen 20 bis 60 nm. Weiter bevorzugt umfassen die erfindungsgemäßen Zubereitungen auf Basis von Mikroemulsion nicht ionische Emulgatoren, vorzugsweise polyethoxylierte Fettalkoholether, ganz besonders bevorzugt C 16-18 Fettalkoholether (20 E.O.).

In einer besonders bevorzugten Ausführungsform umfasst die Mikroemulsion einen C 16-18 Fettalkoholether (20 E.O.) als alleinigen O/W-Emulgator in Kombination mit einem ebenfalls nicht ionischen W/O-Emulgator, vorzugsweise gewählt aus Fettsäure-Glycerin-Estern, ganz besonders bevorzugt Glyceryl Isostearate.

Die erfindungsgemäßen Zubereitungen zeichnen sich weiterhin durch ihre guten sensorischen Eigenschaften verbunden mit einem angenehmen Kühleffekt nach Applikation aus. Dieser Effekt erlaubt den Verzicht auf Ethanol o.ä. Lösemittel, was zu einer sehr guten Hautverträglichkeit insbesondere auch nach der Achselhaar-Rasur führt. Diese Eigenschaft wird durch die insgesamt sehr gute Hautverträglichkeit der Zubereitung unterstützt.

Viele kosmetische Zubereitungen enthalten Parfüm. Diese wiederum umfassen eine Vielzahl von Parfümkomponenten (Riechstoffen) und häufig lipophile Trägermaterialien. Auch hier wurde vielfach ein Problem in polyolefinischen Packmifteln beobachtet. Auch die Parfümöle zeigen Instabilitäten in polyolefinischen Packmitteln. So wurde bei Zubereitungen umfassend Parfümöle umfassend TEC (Triethylcitrat, 2-Hydroxy-1,2,3-propantricarbonsäuretriethylester) in polyolefinischen Packmitteln eine Phasentrennung beobachtet. In Glasbehältnissen wurde diese Instabilität nicht festgestellt. Der Zusatz an Migrationsinhibitoren, insbesondere Paraffinöle, vermindert diese Instabilität ebenso, wie zuvor erläutert.
Bekannt ist jedoch, dass bei Kombination von Paraffinöl mit Parfümölen eigentlich eine verminderte Duftfreisetzung der eingesetzten Parfümöle zu beobachten ist. Überraschend wurde aber neben der Vermeidung der Separation/Phasentrennung auch eine gute Duftfreisetzung beobachtet, wenn anstelle TEC (Triethylcitrat, 2-Hydroxy-1,2,3-propantricarbonsäuretriethylester) Isopropylmyristat (IPM) als Ölkomponente eingesetzt wird. Für eine gute Duftfreisetzung trägt dabei sicher auch die parfümistische Ausgestaltung bei, dennoch wird dies durch den IPM verbessert.

Nun ist aber zu erwarten gewesen, dass auch IPM Migrationsprobleme bereitet.

Erfindungsgemäß ist aber durch den Zusatz von Migrationsinhibitoren, insbesondere Paraffinölen, nicht nur die Migration von IPM verhindert worden, sondern zugleich auch die Duftfreisetzung bei Parfümvorhandensein positiv beeinflusst worden, was so nicht zu erwarten gewesen ist.

Als problematisch hinsichtlich der Migration durch polyolefinische Packmaterialien sind Parfümöle daher ebenso zu betrachten und durch den erfindungsgemäßen Zusatz an Migrationsinhibitoren wird dieses Problem gelöst.
Vorteilhaft ist so der Zusatz von Paraffinölen, Decyloleat und/oder hydrogenated Polydecene zu Zubereitungen umfassend Parfümöle und insbesondere keine weiteren Öle. So sind Beispielzubereitungen 2 und 3 Mikroemulsionen mit Paraffinöl als Migrationsinhibitor und Parfümölen (im Parfüm) ohne weiteren Lipidgehalt.

Insbesondere ist der Parfümbestandteil 2-Hydroxy-1,2,3-propantricarbonsäuretriethylester (TEC) als migrationsfähig identifiziert worden. Bevorzugt ist hier insbesondere der Ersatz von TEC durch IPM in Kombination mit Paraffinölen.
Die Kombination von IPM und Paraffinölen in PP-Packmitteln führt zu stabilen Formulierungen, d.h. Ölmigration und Instabilitäten, z.B. Phasentrennung der Zubereitungen in den PP-Packmitteln, werden verhindert. Ferner wird die Freisetzung von Parfümgeruch durch das Packmittel hindurch unterbunden bzw. minimiert.

Weiterer Vorteil der erfindungsgemäßen Zubereitungen ist, dass sie im Gegensatz zu den in WO 1996028131 A2 und WO 1996028132 A2 beschriebenen Emulsionen zu dessen Herstellung nicht zwingend die Anwendung des sog. PIT-Verfahrens erfordern.

Das Phänomen der Phaseninversionstemperatur (PIT) stellt insbesondere eine Besonderheit bei Emulsionen umfassend nicht-ionische Emulgatoren dar, wie erfindungsgemäß bevorzugt.
Der Begriff der Phaseninversionstemperatur bezieht sich auf eine Phaseninversion (Phasenumkehr), die durch eine Temperaturänderung induziert wird. Nicht-ionische, ethoxilierte Emulgatoren haben die Eigenart, dass sie ihre hydrophilen oder lipophilen Eigenschaften in Abhängigkeit der Temperatur ändern. Damit verbunden ist eine Veränderung ihres HLB-Wertes. Bei der Emulsionsherstellung unter Ausnutzung dieser temperatur-induzierten Phaseninversion wird ein eigentlich hydrophiler (O/W) Emulgator in der Lipidphase aufgeschmolzen. Bedingt durch die Temperaturerhöhung wird der O/W-Emulgator dabei zunehmend lipophiler, so dass sich bei Phasenvereinigung eine W/O-Emulsion mit relativ großen, Emulgator-besetzten Wassertröpfchen bildet. Bei nachfolgender Abkühlung kehrt der Emulgator in seinen ursprünglichen HLB-Wert, entsprechend eines O/W-Emulgators zurück, so dass die zunächst gebildete W/O-Emulsionsstruktur nicht mehr aufrecht erhalten werden kann und somit eine Phasenumkehr erfolgt. Dabei bilden sich ohne einen Homogenisierschritt (mechanischer Energieeintrag) feinstdisperse Systeme aus. Zu charakterisieren ist der Punkt der Phaseninversion durch ein extremes Minimum der Grenzflächenspannung und durch eine sprunghafte Veränderung der elektrischen Leitfähigkeit. So lässt sich mittels Konduktometrie ermitteln, dass die Systeme, wie sie beispielsweise in der WO 1996028131 A2 dargestellt sind, i.d.R. eine Phasenumkehr bei Temperaturen oberhalb 75 °C zeigen. Zur Herstellung dieser Systeme werden Fett- und Wasserphase im Sinne der WO 1996028131 A2 weit über diesen Punkt hinaus erhitzt um ein problemloses Durchlaufen des Phasenumkehrprozesses sicher zu stellen, so dass im Sinne einer sicheren Prozessführung Temperaturen von 85 °C - 90 °C bei Phasenvereinigung angewendet werden.

Diese thermische Belastung ist jedoch kritisch bei der Einarbeitung von Parfümölen und temperaturempfindlichen Wirkstoffen in solche Emulsionen. Hohe Temperaturen bewirken einen Verlust an Kopfnoten im Parfüm, wodurch der olfaktorische Gestaltungsspielraum eingeschränkt wird. Einschlägige Vorschriften erfordern eine entsprechende Anhebung der Flammpunkte der für das PIT-Verfahren eingesetzten Parfümöle, was ebenfalls nachteilig sein kann.

Wünschenswert ist es daher ein Verfahren zur Herstellung von Emulsionen bereit zu stellen, bei dem diese thermische Belastung, insbesondere bei der Herstellung Parfümhaltiger Zubereitungen, vermieden wird.

Es wurde nun überraschend gefunden, dass die Herstellung von feinst-dispersen Emulsionen, wie erfindungsgemäß bevorzugt, nicht zwingend die Anwendung des sogenannten PIT-Verfahrens erfordert.
Vielmehr ist es gelungen, Zubereitungen bei ca. 70 °C herzustellen, was eine Herstellung unterhalb der Phaseninversionstemperatur bedeutet und 15 - 20 °C unterhalb des Verfahrens gemäß WO 1996028131 und WO 1996028132 liegt.
Ferner ergeben sich aufgrund des erfindungsgemäßen Verfahrens erhebliche Vorteile in Hinblick auf Prozesszeiten, da Aufheiz- und Kühlvorgänge erheblich verkürzt werden können. Außerdem wird erfindungsgemäß die Materialbeanspruchung und die Korrosion minimiert.
Besonders vorteilhaft an dieser Herstellung ist die geringere thermische Belastung, was insbesondere die Verwendung von Wirkstoffen und Parfümölen in den erfindungsgemäßen Zubereitungen begünstigt. Bedingt durch die geringeren Herstelltemperaturen können auch die Flammpunkte der eingesetzten Parfümöle erniedrigt werden, was olfaktorische Vorteile bewirkt.
Dies ist insbesondere für die Aufgabenstellung einer stabilen, klaren bis transluzenten Emulsion umfassend Parfümöle förderlich.

Der erfindungsgemäße Herstellprozess umfasst ein Verfahren zur Herstellung von Emulsionen mit einer Tröpfchengröße kleiner 100 nm, insbesondere im Bereich von etwa 20 bis 60 nm, indem die Lipidphase und Wasserphase der Emulsion getrennt durchmischt und auf eine Temperatur unterhalb der Phaseninversionstemperatur erwärmt werden, anschließend werden die Öl- und Wasserphasen vereinigt und auf Raumtemperatur abgekühlt.

Die Herstellung der erfindungsgemäßen Systeme erfolgt nach einem Verfahren der Emulsionsherstellung, ähnlich dem sogenannten "heiß/heiß"-Verfahren.
Bevorzugt wird die Fettphase auf ca. 65 bis 70 °C erhitzt und dabei vollständig und homogen aufgeschmolzen und im Anschluss mit der ebenfalls ca. 65 bis 70 °C heißen Wasserphase vereinigt. Die Fettphase enthält neben den flüssigen oder halbfesten lipophilen Bestandteilen (einschließlich des Parfümöles) auch die Emulgatoren und sonstige amphiphile Bestandteile. Die Wasserphase besteht aus allen wasserlöslichen Bestandteilen und wird auf die gleiche Temperatur gebracht wie die Fettphase und im Anschluss zu dieser bei guter Durchmischung zugegeben. Bei der apparativen Umsetzung im Technikums- oder Produktionsmaßstab kann es vorteilhaft sein, die heiße Wasserphase vorzulegen und die heiße Fettphase unter Sicherstellung guter Durchmischung zuzugeben. D.h. beide Varianten, Ölphase vorlegen und Wasserphase zugeben oder umgekehrt sind erfindungsgemäße Ausführungsvarianten.
Im Anschluss erfolgt eine Abkühlung auf Raumtemperatur, je nach Maßstab ggf. unter Verwendung einer entsprechenden Kühlvorrichtung. Während des gesamten Kühlprozesses ist es vorteilhaft auf eine gute Durchmischung der Emulsion zu achten, wobei aber auf die sonst obligatorische Verwendung eines Homogenisierschrittes ausdrücklich verzichtet werden kann. Es wird demzufolge keine mechanische Energie zur Herstellung der Systeme eingetragen.
Eine homogene Durchmischung sowohl der vorgelegten Öl- und Wasserphasen als auch der Emulsion ist vorteilhaft.
In Hinblick auf die Begrifflichkeit "Nanoemulsion" des Standes der Technik wird erfindungsgemäß auch auf den Einsatz eines Hochdruckhomogenisators verzichtet. Im Stand der Technik werden Mikro- oder Nanoemulsionen durch die Tröpfchengrößen bestimmt, wobei hier keine einheitliche Größenangabe im Stand der Technik festzustellen ist.
Phaseninversionstemperatur Emulsionen (PIT) sind häufig als sogenannte Nanoemulsionen im Stand der Technik beschrieben.
Erfindungsgemäße Emulsionen werden deshalb allein durch ihre Tröpfchengröße unterhalb von 100 nm, insbesondere im Bereich von von 20 bis 60 nm, definiert.

Viele kosmetische Zubereitungen enthalten Parfüme. Diese wiederum umfassen eine Vielzahl von Parfümkomponenten (Riechstoffen) und häufig lipophile Trägermaterialien. Erfindungsgemäß können diese ungeachtet ihrer Temperaturempfindlichkeit nun besser und stabiler in Emulsionen eingearbeitet werden und es ergeben sich stabile Emulsionen mit einer Tröpfchengröße unterhalb von 100 nm.

In Beispiel 1 ist die Ölphase, umfassend alle amphiphilen Substanzen und Lipide sowie Parfüm (Paraffinöl, Octyldodecanol, Isoceteth-20, Glyceryl Isostearate, PEG-150 Distearate und Parfüm) auf 65°C bis 70°C erwärmt worden, parallel ist die Wasserphase, umfassend Wasser, Feuchthaltemittel und AT-Wirkstoff ebenfalls auf 65° bis 70°C erwärmt worden. Beide Phasen werden homogen vermischt und miteinander vereinigt indem man die Wasserphase zur vorgelegten Fettphase gibt. Anschließend lässt man die Zubereitung bei gleichmäßiger Durchmischung abkühlen.
Es ergibt sich eine transluzente, feinst-disperse Emulsion mit einer engen Tröpfchengrößenverteilung (geringe Polydispersität) und einer mittleren Tröpfengröße von 50 nm. Die Bestimmung der Tröpfchengröße erfolgte mittels dynamischer Lichtstreuung. Dazu wurden die Proben mit Wasser verdünnt. Die Detektion erfolgte unter einem Winkel von 90°. Die Zubereitung weißt eine sehr gute physikalische Langzeitstabilität auf.

Zum Vergleich wurde die Zubereitung des Beispiel 1 mit 3 % Dicaprylyl Ether an Stelle von Paraffinöl untersucht, die ebenfalls bei 65 °C, also unterhalb der PIT hergestellt wurde.
Die Tröpfchengröße der hergestellten Emulsion lag nun bei ca. 90 nm und das System war polydispers. Es kam im Laufe der Lagerung zur physikalischen Instabilität.

Somit zeigt sich erneut, dass der Zusatz an Migrationsinhibitoren, wie insbesondere Paraffinöl, die Stabilität von Mikroemulsionen erhöhen vermag.
Der Zusatz der Migrationsinhibitoren im erfindungsgemäßen Verfahren zur Herstellung von Emulsionen, insbesondere mit Tröpfchengrößen unter 100 nm, ist damit predestiniert.

Die erfindungsgemäßen Zubereitungen können kosmetische Hilfsstoffe enthalten, wie sie üblicherweise in solchen Zubereitungen verwendet werden, z.B. Konservierungsmittel, Bakterizide, UV-Filter, Antioxidantien, wasserlösliche Vitamine, Mineralstoffe, suspendierte Festkörperpartikel, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Alkohole, Polyole, Polymere, Schaumstabilisatoren oder Silikonderivate.

Neben anderen in der Kosmetik üblichen Hilfs- und Zusatzstoffen, können die erfindungsgemäßen Zubereitungen selbstverständlich auch Pflanzenextrakte, insbesondere auch solche auf einer lipophilen Trägermatrix beinhalten. Derartige Extrakte können z.B. durch Pressen von Fruchtfleisch oder Samen, oder durch die Verwendung zusätzlicher Extraktions- bzw. Trägermittel an Teilen der entsprechenden Pflanze oder deren Frucht bereit gestellt werden. Der Zusatz solcher lipophilen Substanzen stellt erfindungsgemäß keine Verwendung *zusätzlicher* Lipide dar.

Polyolefinische Packmittel umfassen erfindungsgemäß Behältnisse, insbesondere Tuben, Flaschen, Roll-ons, die aus Polyolefinen aufgebaut sind oder zumindest Polyolefine beinhalten. Auch Roll-on Verpackungen deren Kugel und/oder Fitment aus PE bzw. PP aufgebaut sind, sind als erfindungsgemäße polyolefinische Packmittel zu verstehen. Ebenso sind unter polyolefischen Packmitteln alldiejenigen Packmittel zu verstehen, die vollständig aus Polyolefinen bestehen oder nur Teile aus Polyolefinen, insbesondere Polypropylen, umfassen, die in Kontakt mit der Zubereitungen stehen können.

Bevorzugt wird als Polyolefin Polypropylen gewählt, insbesondere sind die erfindungsgemäßen Packmittel aus Polypropylen (PP) aufgebaut.

Bekannte Kunststoffe wie PET, PETE, Polyethylenterephthalate sind Polyester aus der Gruppe der Polyalkylenterephthalate und sind erfindungsgemäß keine polyolefinischen Packmittel.

Die folgenden Vergleichsuntersuchungen belegen die Migrationsinhibierung von Lipiden in/durch Polyolefin-, insbesondere PP-haltige Verpackungen.

Es wurden dazu Polypropylen-haltige Prüfkörper in Zubereitungen A und B gelagert, die sich wie folgt zusammensetzen:

| Vergleichszubereitungen | A | B |
|---|---|---|
| Aqua | ad 100 | ad 100 |
| Aluminum Chlorohydrate (50 %_{aq}) | 0 | 20 |
| Butylene Glycol oder Glycerin | 0-3 | 0-3 |
| Isoceteth-20 | 3,0 - 5,5 | 3,0 - 5,5 |
| Glyceryl Isostearate | 1,0 - 3,0 | 1,0 - 3,0 |
| PEG-150 Distearate | 0,5 - 1,0 | 0,5 - 1,0 |
| Lipid C | 3 | 3 |
| Migrationsinhibitor D | 3 | 3 |
| Parfüm | 1 | 1 |

B stellt eine typische Mikroemulsion mit Antitranspirantwirkstoff (AT) dar, A ist eine Mikroemulsion ohne AT-wirkstoff.
Lipid C wird jeweils gewählt aus C13-16 Isoparaffin, Dicaprylyl Ether, Dicaprylyl Carbonate, Diethylhexyl Carbonate, Coco-Caprylate/Caprate, C12-15 Alkyl Benzoate, Ethylhexyl Cocoate, Isopropyl Stearate, Octyldodecanol, Isopropyl Palmitate oder Isopropyl Myristate zu einem Anteil von 3 Gew.%.
Die Migrationsinhibitoren D, Paraffinöl, Decyl Oleat und/oder hydrogenated Polydecene, werden den Vergleichszubereitungen zu einem Anteil von 3 Gew.% zugesetzt (X) bzw. sie sind nicht zugesetzt (Y). Die Emulgatoren werden im angegebenen Bereich je nach zugesetztem Lipid gewählt um entsprechende Mikroemulsionen zu erhalten, was dem im Bereich Kosmetik erfahrenen Fachmann bekannt ist. So ist beispielsweise Zubereitung B mit 2,7 Gew.% Glycerin, 3 Gew.% Paraffinöl und 3 Gew.% Octyldodecanol entsprechend Beispielzubereitung 1 zu formulieren.

Getestet wurde die Migration indem die Gewichtszunahme eines PP-Prüfkörpers nach 28 Tagen Lagerung bei 40°C in den verschiedenen Zubereitungen gemessen wurde.
Auf diese Art ist es möglich, quantitativ das Penetrationsverhalten von Stoffen in Verpackungsmaterialien bzw. das Absorptionsvermögen von Verpackungsmaterialien zu bestimmen. Es wurden Stanzlinge, mit einem Durchmesser von 5mm, hergestellt und verwendet. Diese Packmittelscheiben wurden anschließend in einem Glas, gefüllt mit der jeweiligen Zubereitung, bei der entsprechenden Temperatur von 40°C gelagert. Die Gewichtsänderungen werden auf das Startgewicht bezogen (Nullmessung = unkontaminiertes Packmittel). Die Bestimmung erfolgte mittels einer Ultramikrowaage (UMX).

Tabelle 1 zeigt die ermittelte Gewichtszunahme des PP-Prüfkörpers der jeweiligen Zubereitungen A und B, die jeweils die aufgeführten Lipide und jeweils die aufgeführten Migrationsinhibitoren umfassten(X) bzw. ohne die Inhibitoren (Y). Ein Unterschied zwischen den Zubereitungen A und B wurde nicht beobachtet.

**Tabelle 1:**

| D | Paraffinöl | Decyl Oleat | hydrogenated Polydecene | ohne |
|---|---|---|---|---|
| Gew.% | X | X | X | Y |
| C13-16 Isoparaffin | 3,1 | 2,5 | 1,7 | 5,3 |
| Dicaprylyl Ether | 2,6 | 2,4 | 1,7 | 5,1 |
| Dicaprylyl Carbonate | 2,5 | 2,4 | 1,6 | 4,9 |
| Diethylhexyl Carbonate | 2,5 | 2,5 | 1,8 | 5,0 |
| Coco-Caprylate/Caprate | 2,0 | 2,0 | 1,6 | 4,4 |
| C12-15 Alkyl Benzoate | 2,3 | 2,3 | 2,0 | 4,5 |
| Ethylhexyl Cocoate | 2,7 | 2,7 | 2,4 | 4,7 |
| Isopropyl Stearate | 2,5 | 2,3 | 2,3 | 4,8 |
| Octyldodecanol (Bsp. 1) | 1,0 | 0,9 | 0,9 | 2,1 |
| Isopropyl Palmitate | 2,9 | 2,7 | 2,7 | 4,8 |
| Isopropyl Myristate | 3,3 | 3,2 | 3,2 | 5,2 |

Der Vergleich zwischen den Zubereitungen einmal mit (X) und einmal ohne (Y) Migrationsinhibitoren zeigt (s. Tabelle 1), dass alle aufgeführten Lipide und alle Migrationsinhibitoren die Migration der Lipide durch PP-haltige Verpackungen vermindern bzw. verhindern.
In gleicher Weise wurde exemplarisch auch die Ölaufnahme von PE-Prüfkörpern untersucht und auch dort wurde die Migration der genannten Lipide in die Prüfkörper festgestellt bzw. die Verminderung dieser Migration durch Zusatz von Paraffinöl, Decyl Oleat und/oder hydrogenated Polydecene.
Damit ist die Verminderung bzw. Vermeidung der Ölmigration in oder durch polyolefinhaltige Verpackungen gezeigt.

Erfindungsgemäß ist daher die Verwendung der Migrationsinhibitoren in Lipid-haltigen Zubereitungen zur Vermeidung der Migration der Lipide in oder durch Packmittel bestehend aus oder umfassend Polyolefine, bevorzugt Polypropylen.
Die Migrationsinhibitoren werden erfindungsgemäß als Zubereitungsbestandteile verstanden, die die Migration von Lipiden in oder durch das polyolefinische Packmittel verhindern.
Als erfindungsgemäße Inhibitoren sind dabei alle Stoffe aufzufassen, die obige Wirkung zeigen.
Erfindungsgemäß sind bevorzugt die Paraffinöle als Migrationsinhibitoren zu nennen, da sie auch die in kosmetischen Zubereitungen häufig zum Einsatz kommenden Parfüme stabilisieren (s. oben).

Nachfolgende Beispiele erläutern die erfindungsgemäßen Produkte. Die Angaben beziehen sich auf Gewichtsanteile, bezogen auf die Gesamtmasse der Zubereitungen.

| Beispiele | 1 | 2 | 3 | 4 |
|---|---|---|---|---|
| Aqua | ad 100 | ad 100 | ad 100 | ad 100 |
| Aluminum Chlorohydrate (50 %_{aq}) | 20 | 25 | 20 | 15 |
| Butylene Glycol | | 3 | 3 | 3 |
| Glycerin | 2,6 | 3 | 3 | 3 |
| Paraffinum Liquidum | 3 | 3 | 3 | 2 |
| Octyldodecanol | 3 | | | |
| C13-16 Isoparaffin | | | | 2 |
| Isoceteth-20 | 4 | 5 | 4,9 | 4,8 |
| Glyceryl Isostearate | 2 | 2,2 | 2,3 | 2,4 |
| PEG-150 Distearate | 0,7 | 0,7 | 0,7 | 0,7 |
| Parfüm | 1 | 1 | 1 | 1 |
| Packmittel | PP-Roll-on | PP-Roll-on | PP-Roll-on | PP-Roll-on |
| Optik | transluzent | transparent | transparent | transparent |
| Stabilität | 2 Monate | mind. 2 Monate RT und B40 | mind. 2 Monate RT und B40 | mind. 2 Monate RT und B40 |

| | | | |
|---|---|---|---|
| Beispiele | 5 | 6 | 7 |
| Aqua | ad 100 | ad 100 | ad 100 |
| Aluminum Chlorohydrate (50 %_{aq}) | 25 | 25 | |
| Butylene Glycol | 3 | 3 | 3 |
| Decyl Oleate | 3 | 3 | |
| Hydrogenated Polydecene | | | 3 |
| Diethylhexyl Carbonate | 1 | 1 | 2 |
| Isoceteth-20 | 6 | 5,6 | 5,4 |
| Glyceryl Isostearate | 1,2 | 1,6 | 1,8 |
| PEG-150 Distearate | 0,7 | 0,7 | 0,7 |
| Parfüm | 1 | 1 | 1 |
| Packmittel | PP-Roll-on | PP-Roll-on | PP-Roll-on |

### Beispiel 8 - Feststellung der Migration

Zubereitungen mit Dicaprylyl Ether und ohne Migrationsinhibitoren wurden in Polypropylenflaschen (Roll-on) gelagert.
Die quantitative Bestimmung des Lipidgehaltes im Füllgut nach Lagerung ergab:
c(Dicaprylyl Ether)ₜ₀ = 3,0 %
c(Dicaprylyl Ether)ₜ₁ = 0,5 %
t1 = 6 Monate, 40 °C gelagert
D.h. das Füllgut hat nach 6 Monaten einen Ölverlust von 2,5%.
Dicaprylyl Ether konnte im Packmittel nachgewiesen werden.
Das Füllgut umfasste die Zubereitung mit (Gew.%):

| Aqua | ad 100 |
|---|---|
| Glyceryl Isostearate | 2,4 |
| Butylene Glycol | 3 |
| Aluminum Chlorohydrate | 20 |
| Dicaprylyl Ether | 3 |
| PEG-150 Distearate | 0,7 |
| Isoceteth-20 | 4,8 |
| Parfüm | 1 |

## Patentansprüche

1. Produkt umfassend Packmittel bestehend aus oder umfassend Polyolefine und im Packmittel enthaltende Emulsionszubereitung umfassend mindestens einen Migrationsinhibitor und mindestens ein Lipid gewählt werden aus der Gruppe der Parfümöle, C13-16 Isoparaffin, Dicaprylyl Ether, Dicaprylyl Carbonate, Diethylhexyl Carbonate, Coco-Caprylate/Caprate, C12-15 Alkyl Benzoate, Ethylhexyl Cocoate, Isopropyl Stearate, Octyldodecanol, Isopropyl Palmitate und/oder Isopropyl Myristate, , **dadurch gekennzeichnet, dass** der Migrationsinhibitor gewählt wird aus der Gruppe Paraffinöl, Decyl Oleat und/oder hydrogenated Polydecene.

2. Produkt nach Anspruch 2 **dadurch gekennzeichnet, dass** der Migrationsinhibitor Paraffinöl ist.

3. Produkt nach Anspruch 2 **dadurch gekennzeichnet, dass** das Parfümöl 2-Hydroxy-1,2,3-propantricarbonsäuretriethylester, Dipropylenglycol und/oder Hexylsalicylat. umfasst.

4. Produkt nach Anspruch 1 **dadurch gekennzeichnet, dass** die Zubereitung eine Mikroemulsion ist.

5. Produkt nach Anspruch 4 **dadurch gekennzeichnet, dass** die Mikroemulsion eine Tröfpchengröße kleiner 100 nm, insbesondere im Bereich von 20 bis 60 nm, aufweist.

6. Produkt nach Anspruch 4 oder 5 **dadurch gekennzeichnet, dass** die Zubereitung nicht ionische Emulgatoren, insbesondere polyethoxylierte Fettalkoholether, ganz besonders bevorzugt C 16-18 Fettalkoholether, umfasst.

7. Produkt nach Anspruch 6 **dadurch gekennzeichnet, dass** C 16-18 Fettalkoholether als alleinigen O/W-Emulgator enthalten ist.

8. Produkt nach Anspruch 7 **dadurch gekennzeichnet, dass** der C 16-18 Fettalkoholether in Kombination mit einem nicht ionischen W/O-Emulgator, vorzugsweise gewählt aus Fettsäure-Glycerin-Estern, ganz besonders bevorzugt Glyceryl Isostearate, enthalten ist.

9. Produkt nach einem der Ansprüche 4 bis 8 **dadurch gekennzeichnet, dass** neben ein oder mehreren Migrationsinhibitoren als Lipidkomponente der Zubereitung nur ein oder mehrere Parfümöle und/oder Isopropyl Myristate enthalten sind.

10. Produkt nach Anspruch 9 **dadurch gekennzeichnet, dass** neben ein oder mehreren Migrationsinhibitoren als Lipidkomponente der Zubereitung nur ein oder mehrere Parfümöle enthalten sind.

11. Produkt nach einem der vorstehenden Ansprüche **dadurch gekennzeichnet, dass** das Packmittel auf der mit der Zubereitung in Kontakt tretenden Fläche nicht beschichtet oder laminiert ist.

12. Produkt nach einem der vorstehenden Ansprüche **dadurch gekennzeichnet, dass** das Polyolefin Polypropylen ist.

13. Produkt nach einem der vorstehenden Ansprüche **dadurch gekennzeichnet, dass** es sich um eine Antitranspirant- und/oder Deodorantzubereitung in einer Roll-on Verpackung handelt.

14. Verwendung von Migrationsinhibitoren in Lipid-haltigen Zubereitungen zur Verminderung oder Vermeidung der Migration der Lipide gewählt aus der Gruppe C13-16 Isoparaffin, Dicaprylyl Ether, Dicaprylyl Carbonate, Diethylhexyl Carbonate, Coco-Caprylate/Caprate, C12-15 Alkyl Benzoate, Ethylhexyl Cocoate, Isopropyl Stearate, Octyldodecanol, Isopropyl Palmitate, Isopropyl Myristate und/oder Parfümölen, insbesondere umfassend Triethyl Citrate, Dipropylenglycol und/oder Hexylsalicylat, in oder durch Packmittel bestehend aus oder umfassend Polyolefine, **dadurch gekennzeichnet, dass** der Migrationsinhibitor gewählt wird aus der Gruppe Paraffinöl, Decyl Oleat und/oder hydrogenated Polydecene.

15. Verwendung nach Anspruch 14 **dadurch gekennzeichnet, dass** das Polyolefin Polypropylen ist.

16. Verfahren zur Herstellung von Emulsionen mit einer Tröpfchengröße kleiner 100 nm, insbesondere im Bereich von etwa 20 bis 60 nm, indem die Lipidphase und Wasserphase der Emulsion getrennt durchmischt und auf eine Temperatur unterhalb der Phaseninversionstemperatur erwärmt, anschließend die Öl- und Wasserphasen vereinigt, vermischt und auf Raumtemperatur abgekühlt werden, **dadurch gekennzeichnet, dass** die Lipidphase ein oder mehrere Migrationsinhibitoren gewählt aus der Gruppe Paraffinöl, Decyl Oleat und/oder hydrogenated Polydecene umfasst.

17. Verfahren nach Anspruch 16 **dadurch gekennzeichnet, dass** die Lipidphase ein oder mehrere Lipide gewählt aus der Gruppe C13-16 Isoparaffin, Dicaprylyl Ether, Dicaprylyl Carbonate, Diethylhexyl Carbonate, Coco-Caprylate/Caprate, C12-15 Alkyl Benzoate, Ethylhexyl Cocoate, Isopropyl Stearate, Octyldodecanol, Isopropyl Palmitate, Isopropyl Myristate und/oder Parfümöle, insbesondere umfassend Triethyl Citrate, Dipropylenglycol und/oder Hexylsalicylat, umfasst.

18. Verfahren nach Anspruch 16 oder 17 **dadurch gekennzeichnet, dass** die Temperatur maximal 70° C beträgt.

## Claims

1. Product comprising packaging consisting of or comprising polyolefins and emulsion preparation, contained in the packaging, comprising at least one migration inhibitor and at least one lipid selected from the group of the perfume oils, C13-16 Isoparaffin, Dicaprylyl Ether, Dicaprylyl Carbonate, Diethylhexyl Carbonate, Coco-Caprylate/Caprate, C12-15 Alkyl Benzoate, Ethylhexyl Cocoate, Isopropyl Stearate, Octyldodecanol, Isopropyl Palmitate and/or Isopropyl Myristate, **characterized in that** the migration inhibitor is selected from the group paraffin oil, decyl oleate and/or hydrogenated polydecene.

2. Product according to Claim 1, **characterized in that** the migration inhibitor is paraffin oil.

3. Product according to Claim 2, **characterized in that** the perfume oil comprises triethyl 2-hydroxy-1,2,3-propanetricarboxylate, dipropylene glycol and/or hexyl salicylate.

4. Product according to Claim 1, **characterized in that** the preparation is a microemulsion.

5. Product according to Claim 4, **characterized in that** the microemulsion has a droplet size less than 100 nm, in particular in the range from 20 to 60 nm.

6. Product according to Claim 4 or 5, **characterized in that** the preparation comprises nonionic emulsifiers, in particular polyethoxylated fatty alcohol ethers, very particularly preferably C 16-18 fatty alcohol ethers.

7. Product according to Claim 6, **characterized in that** C 16-18 fatty alcohol ether is present as the sole O/W emulsifier.

8. Product according to Claim 7, **characterized in that** the C 16-18 fatty alcohol ether is present in combination with a nonionic W/O emulsifier, preferably selected from fatty acid glycerol esters, very particularly preferably glyceryl isostearate.

9. Product according to one of Claims 4 to 8, **characterized in that**, besides one or more migration inhibitors, as lipid component of the preparation, only one or more perfume oils and/or isopropyl myristate are present.

10. Product according to Claim 9, **characterized in that**, besides one or more migration inhibitors, as lipid component of the preparation, only one or more perfume oils are present.

11. Product according to one of the preceding claims, **characterized in that** the packaging is not coated or laminated on the surface which comes into contact with the preparation.

12. Product according to one of the preceding claims, **characterized in that** the polyolefin is polypropylene.

13. Product according to one of the preceding claims, **characterized in that** it is an antiperspirant and/or deodorant preparation in a roll-on packaging.

14. Use of migration inhibitors in lipid-containing preparations for reducing or avoiding the migration of the lipids selected from the group C13-16 Isoparaffin, Dicaprylyl Ether, Dicaprylyl Carbonate, Diethylhexyl Carbonate, Coco-Caprylate/Caprate, C12-15 Alkyl Benzoate, Ethylhexyl Cocoate, Isopropyl Stearate, Octyldodecanol, Isopropyl Palmitate, Isopropyl Myristate and/or perfume oils, in particular comprising triethyl citrate, dipropylene glycol and/or hexyl salicylate, into or through packaging consisting of or comprising polyolefins, **characterized in that** the migration inhibitor is selected from the group paraffin oil, decyl oleate and/or hydrogenated polydecene.

15. Use according to Claim 14, **characterized in that** the polyolefin is polypropylene.

16. Process for the preparation of emulsions with a droplet size less than 100 nm, in particular in the range from about 20 to 60 nm, by separately mixing the lipid phase and the water phase of the emulsion and heating them to a temperature below the phase inversion temperature, then combining the oil and water phases, mixing them and cooling them to room temperature, **characterized in that** the lipid phase comprises one or more migration inhibitors selected from the group paraffin oil, decyl oleate and/or hydrogenated polydecene.

17. Process according to Claim 16, **characterized in that** the lipid phase comprises one or more lipids selected from the group C13-16 Isoparaffin, Dicaprylyl Ether, Dicaprylyl Carbonate, Diethylhexyl Carbonate, Coco-Caprylate/Caprate, C12-15 Alkyl Benzoate, Ethylhexyl Cocoate, Isopropyl Stearate, Octyldodecanol, Isopropyl Palmitate, Isopropyl Myristate and/or perfume oils, in particular comprising triethyl citrate, dipropylene glycol and/or hexyl salicylate.

18. Process according to Claim 16 or 17, **characterized in that** the temperature is at most 70°C.

## Revendications

1. Produit comprenant un matériau d'emballage consistant en ou comprenant des polyoléfines et, contenue dans le matériau d'emballage, une préparation en émulsion comprenant au moins un inhibiteur de migration et au moins un lipide choisi dans le groupe des huiles essentielles, C13-16 isoparaffin, dicaprylyl ether, dicaprylyl carbonate, diethylhexyl carbonate, coco-caprylate/caprate, C12-15 alkyl benzoate, ethylhexyl cocoate, isopropyl stearate, octyldodecanol, isopropyl palmitate et/ou isopropyl myristate, **caractérisé en ce que** l'inhibiteur de migration est choisi dans le groupe constitué par une huile de paraffine, le decyl oleate et/ou l'hydrogenated polydecene.

2. Produit selon la revendication 1, **caractérisé en ce que** l'inhibiteur de migration est l'huile de paraffine.

3. Produit selon la revendication 2, **caractérisé en ce que** l'huile essentielle comprend le 2-hydroxy-1,2,3-propanetricarboxylate de triéthyle, le dipropylèneglycol et/ou le salicylate d'hexyle.

4. Produit selon la revendication 1, **caractérisé en ce que** la préparation est une microémulsion.

5. Produit selon la revendication 4, **caractérisé en ce que** la microémulsion présente une taille de gouttelettes inférieure à 100 nm, en particulier dans la plage de 20 à 60 nm.

6. Produit selon la revendication 4 ou 5, **caractérisé en ce que** la préparation comprend des émulsifiants non ironiques, en particulier des éthers d'alcools gras polyéthoxylés, de façon tout particulièrement préférée des éthers d'alcools gras en C₁₆-C₁₈.

7. Produit selon la revendication 6, **caractérisé en ce qu'**un éther d'alcool gras en C₁₆-C₁₈ est contenu en tant que seul émulsifiant H/E.

8. Produit selon la revendication 7, **caractérisé en ce que** l'éther d'alcool gras en C₁₆-C₁₈ est contenu en association avec un émulsifiant E/H non ionique, de préférence choisi parmi les esters d'acides gras avec le glycérol, de façon tout particulièrement préférée le glyceryl isostearate.

9. Produit selon l'une quelconque des revendications 4 à 8, **caractérisé en ce qu'**en plus d'un ou plusieurs inhibiteurs de migration la préparation ne contient en tant que composant lipidique que de l'isopropyl myristate et/ou une ou plusieurs huiles essentielles.

10. Produit selon la revendication 9, **caractérisé en ce qu'**en plus d'un ou plusieurs inhibiteurs de migration la préparation ne contient en tant que composant lipidique qu'une ou plusieurs huiles essentielles.

11. Produit selon l'une quelconque des revendications précédentes, **caractérisé en ce que** sur la surface entrant en contact avec la préparation le matériau d'emballage n'est pas stratifié ou revêtu.

12. Produit selon l'une quelconque des revendications précédentes, **caractérisé en ce que** la polyoléfine est le polypropylène.

13. Produit selon l'une quelconque des revendications précédentes, **caractérisé en ce qu'**il s'agit d'une préparation antisudorale et/ou déodorante dans un emballage roll-on.

14. Utilisation d'inhibiteurs de migration dans des préparations contenant des lipides, pour la réduction ou l'évitement de la migration des lipides choisis dans le groupe constitué par C13-16 isoparaffin, dicaprylyl ether, dicaprylyl carbonate, diethylhexyl carbonate, coco-caprylate/caprate, C12-15 alkyl benzoate, ethylhexyl cocoate, isopropyl stearate, octyldodecanol, isopropyl palmitate, isopropyl myristate et/ou des huiles essentielles, comprenant en particulier le triethyl citrate, le dipropyléneglycol et/ou le salicylate d'hexyle, dans ou à travers un matériau d'emballage consistant en ou comprenant des polyoléfines, **caractérisée en ce que** l'inhibiteur de migration est choisi dans le groupe constitué par une huile de paraffine, le decyl oleate et/ou l'hydrogenated polydecene.

15. Utilisation selon la revendication 14, **caractérisée en ce que** la polyoléfine est le polypropylène.

16. Procédé pour la préparation d'émulsions ayant une taille de gouttelette inférieure à 100 nm, en particulier dans la plage d'environ 20 à 60 nm, par homogénéisation, séparément, de la phase lipidique et de la phase aqueuse, de l'émulsion et chauffage à une température inférieure à la température d'inversion de phases, ensuite réunion des phases huileuse et aqueuse, mélange et refroidissement jusqu'à la température ambiante, **caractérisé en ce que** la phase lipidique comprend un ou plusieurs inhibiteurs de migration choisis dans le groupe constitué par une huile de paraffine, le decyl oleate et/ou l'hydrogenated polydecene.

17. Procédé selon la revendication 16, **caractérisé en ce que** la phase lipidique comprend un ou plusieurs lipides choisis dans le groupe constitué par C13-16 isoparaffin, dicaprylyl ether, dicaprylyl carbonate, diethylhexyl carbonate, coco-caprylate/caprate, C12-15 alkyl benzoate, ethylhexyl cocoate, isopropyl stearate, octyldodecanol, isopropyl palmitate, isopropyl myristate et/ou des huiles essentielles, comprenant en particulier le triethyl citrate, le dipropylèneglycol et/ou le salicylate d'hexyle.

18. Procédé selon la revendication 16 ou 17, **caractérisé en ce que** la température est au maximum de 70 °C.
